Europäisches Patentamt

European Patent Office

Office européen des brevets

(19)

(11) Publication number: **0 320 271**
**A2**

# EUROPEAN PATENT APPLICATION

(12)

(21) Application number: **88311650.1**

(22) Date of filing: **08.12.88**

(51) Int. Cl.⁴: **A61K 31/10**

The title of the invention has been amended (Guidelines for Examination in the EPO, A-III, 7.3).

(30) Priority: **08.12.87 GB 8728625**
**04.01.88 US 140352**

(43) Date of publication of application:
**14.06.89 Bulletin 89/24**

(84) Designated Contracting States:
**AT BE CH DE ES FR GB GR IT LI LU NL SE**

(71) Applicant: **RHODERTON CORPORATION N.V.**
**De Ruyterkade 58A**
**Willemstad Curacao(AN)**

(72) Inventor: **Vich I Pascuchi, Josep Maria**
**Carrer Major 33 Appartment 7**
**E-08960 Sant Just des Vern(ES)**

(74) Representative: **Woods, Geoffrey Corlett et al**
**J.A. KEMP & CO. 14 South Square Gray's Inn**
**London WC1R 5EU(GB)**

(54) **Dimethylsulfoxide and analogues as anti-retroviral agents.**

(57) A compound of formula (I):

$(R)_2 S(O)_n$    (I)

where R is methyl or ethyl and n is 1 or 2, particularly dimethylsulphoxide, is useful in the treatment of a retroviral infection such as HIV-1.

EP 0 320 271 A2

## ANTI-VIRAL AGENT

The present invention relates to the treatment of human or animal infections attributable to a retrovirus.

Dimethylsulphoxide (DMSO) is a known compound, a colourless oily liquid, for which a variety of physiologic properties have been described. For example, it has local analgesic and anti-inflammatory effects, is bacteriostatic, acts as a diuretic inducing a negative salt balance, has a tranquilising effect and potentiates the effects of other compounds. It is also an antianoxic and has been known to increase the hypoxic survival of rats. Furthermore, it appears to have therapeutic efficacy in the treatment of mental illnesses and rheumatic diseases.

A few investigations into the anti-viral properties of DMSO have been carried out. Remission of crinkle strawberry virus symptoms in Fragaria vesca resulted from spraying infected plants with 500 ppm DMSO containing 500 ppm of 6-mercaptopurine or 100 ppm of 6-methylpurine. Suppression of symptoms and a change in the syndromes of peach mosaic (PMV) and necrotic ringspot viruses (NRSV) resulted from injection of peach trees with DMSO. However, very mild forms of the viruses were subsequently found in the trees. Local lesion symptoms of tobacco mosaic virus (TMV) in several hosts were reduced by mixing 0.05 to 1% DMSO with virus before innoculation (The Sulphur Institute Journal, Autumn 1969, 2-6).

The effect of DMSO on the infectivity of four RNA and two DNA viruses has also been studied (Chan and Gadebusch, Applied Microbiology, vol.16,no.10,1625-1626, 1968). At a concentration of 80% in buffered saline DMSO inactivated the infectivity of every virus tested. However, experiments designed to study the chemotherapeutic value of DMSO showed the compound to possess no beneficial effect when administered parenterally to mice infected with influenza A (PR-8) virus or Semliki Forest virus.

The stabilisation of enveloped viruses by DMSO has been studied (Wallis and Melnick, J. Virol. vol. 2, no. 9,953-954, 1968). These studies showed that concentrations of DMSO as low as 5% effectively protected the enveloped viruses under test against the trauma of freezing, i.e. the viruses were not inactivated by freezing. There was no suggestion that DMSO could be used therapeutically to combat infections attributable to enveloped viruses, particularly retroviruses.

It has now been discovered that DMSO and close analogues are effective in treating infections attributable to a retrovirus. Accordingly the present invention provides for the use of a compound of formula (I):

$(R)_2S(O)_n$

wherein R is methyl or ethyl and n is 1 or 2, in the preparation of a medicament for use in the treatment of an infection attributable to a retrovirus.

Preferably, the compound of formula (I) is DMSO.

The retrovirus against which a compound of formula (I) may be used may be HTLV-1, HTLV-2 or a Lentivirus such as HIV-1, HIV-2, SIV or medi-visna.

The compound of formula (I) is typically formulated as a pharmaceutical composition further comprising a pharmaceutically acceptable carrier or diluent. Preferred diluents are physiologically acceptable solvents such as sterile water (Water for Injections) and physiological saline. The compounds in which n is 2 are solid at room temperature. They may therefore be initially formulated in solid form with a carrier and then dissolved in a diluent immediately prior to use. Compositions may comprise from 0.5 to 100%, for example up to 95%, of a compound of formula (I). Preferred are solutions for parenteral administration comprising from 0.2% to 20%, more preferably from 1 to 5% such as from 1 to 3%, of a compound of formula (I). Percentages are by volume when n is 1 or by weight when n is 2.

The compounds of formula (I) are useful in combating an infection attributable to a retrovirus in humans or animals, especially in humans. They may in particular be used to treat AIDS or AIDS-related complex (ARC). The compounds are generally administered parenterally by injection, for example intravenously, or orally. A therapeutically effective amount is given. The amount of compound administered will depend upon a variety of factors including the patient under treatment and the severity of the infection. Typically, a dose of from 1 to 10 and preferably from 2.5 to 7.5 ml in the case of a compound of formula (I) in which n is 1 or g in the case of a compound of formula (I) in which n is 2 may be administered parenterally, especially intravenously, or orally. A dose may be given once per day for 5 to 10 days, once or twice per month.

The following Examples illustrate the invention. The LDV/5 cell line employed in Examples 1 and 2 is a clone of the LDV/7 cell line described in GB-A-1592954.

Example 1: DMSO in vitro against HIV-1

PROCEDURE:

4 aliquots of $1 \times 10^6$ LDV/5 cells were infected with 0.5 ml of HIV-1 suspended in RPMI without serum at 37°C (5% by volume $CO_2$) for 2 hours. Then, without removing the viral inoculum, the samples were transferred to 25 ml bottles and supplied with 6 ml of RPMI + 10% by volume foetal calf serum (FCS).

IMMUNOFLUORESCENCE RESULTS:

Cells were fixed with methanol/acetone 50/50 by volume at -20°C and processed for indirect immuofluorescence in suspension. Monoclonal antibodies M1 and M2 were used, which were directed against p17 and p24 viral proteins of HIV-1. The fluorescein isothiocyanate conjugated antibody (FITC) used was goat-antimouse with specificity for Fc fragments. The immunofluorescence positive values represent the figures after subtraction of the fluorescence background, which in no case was higher than 2.3%.

RESULTS: M1 and M2, monoclonal antibodies directed against HIV-1 p17 and p24 proteins

A) LDV/5 cells infected with HIV-1 (control)

| M1 | M2 | Days post infection |
|---|---|---|
| 6.7% | 8.2% | 14 days |
| 11.6% | 3.3% | 26 days |
| 2.4% | 5.7% | 42 days |

B) LDV/5 cells infected with HIV-1, then transferred to medium containing 2% by volume DMSO, 3 days after infection

| M1 | M2 | Days post infection |
|---|---|---|
| 0.3% | 0% | 14 days |
| 0% | 0.3% | 26 days |
| 0.3% | 0.5% | 42 days |

C) LDV/5 cells incubated in 2% by volume DMSO, 5 days before infection, then infected in medium without DMSO, and subsequently transferred to medium with 2% by volume DMSO

| M1 | M2 | Days post infection |
|---|---|---|
| 0.3% | 0.6% | 14 days |
| 0.8% | 0% | 26 days |

In this bottle, the cells started to die 3 days after infection. By day 24, all cells were dead (due to DMSO toxicity, not to virus-induced cell lysis).

D) LDV/5 cells incubated in 2% by volume DMSO for 5 days before infection, then infected in absence of DMSO for two hours and, after washing, they were resuspended in normal RPMI + 10% by volume FCS

3

| M1 | M2 | Days post infection |
|------|------|-----------------|
| 4.2% | 2.7% | 14 days |
| 3.3% | 6.1% | 26 days |
| 2.0% | 5.0% | 42 days |

Example 2: DMSO in vitro against SIV

LDV/5 cells were infected with SIV in the same way as for HIV-1 in Example 1. Reverse transcriptase activity was determined on day 18 and on day 25 following the infection.

| RESULTS (cpm) | | | |
|--------|-----------------|-----------|-----------|
|  | Control Culture | Culture 1 | Culture 2 |
| Day 18 | 68,450 | 6,506 | 54,562 |
| Day 25 | 14,380 | 4,380 | 6,456 |

Culture 1: cells were continuously incubated with 2% by volume DMSO, 3 days after viral infection.

Culture 2: cells were incubated with 2% by volume DMSO, 5 days before infection, but only until the day of infection. DMSO was then removed, and the cells were cultured in normal medium.

Example 3: Treatment of a patient infected with HIV-1

This Example relates to the treatment of a male homosexual suffering from AIDS, JFL, aged 36 years. He was diagnosed serum HIV-1 positive and presented asthenia, weight loss, pulmonary tuberculosis and pneumocystosis.

The patient apparently stabilised for four months under correctional treatment, at which time a Kaposi syndrome appeared, this being treated with interferon. The general condition deteriorated: anorexia - loss in weight of nine kilos - but neither fever, nor swollen lymph glands, nor adenopathy nor intestinal problems. However, the haemogram showed very substantial anaemia, 2.5 M red cells and haemoglobin level at 7.5%, ESR 93.127; leucocytes: 2,400 white (34% lymphocytes), platelets 200,000.

There was buccal and digestive candidiasis, as well as a digestive staphylococcal infection treated with injectable delayed-release Extencilline every 15 days. Then neurological disorders appeared with temporospatial disorientation and complete anterograde amnesia. Eventually, delirium appeared with hallucinations, flight of ideas and discordance. This picture was completed with speech disorders, and a neuromuscular atony rendering the patient bed-ridden.

At that time an injection of 5 ml of DMSO in 250 cc of isotonic saline was administered (2 ml/minute). This injection did not cause the appearance of any further sign. Only the characteristic odour appeared, lasting 24 hours.

48 hours later, the patient emerged from the coma, and on the next day the consciousness level returned to normal, enabling the patient to get up.

It was then observed that the patient had complete amnesia for the last twenty days until consciousness returned to normal. Memory, vigilance and the field of consciousness remained normal and stable. On that day, the patient returned home and received a second injection of DMSO under the same conditions as the first. Only a febricula of 38° C persisted, in the evening, and a dry irritation cough.

Example 4: DMSO in vitro against HIV-1

The effect of DMSO on HIV-1 infection of LDV/7 cells was studied. 4 aliquots of $1 \times 10^6$ LDV/7 cells were infected with 0.5 ml of HIV-1 suspended in RPMI without serum at 37° C (5% by volume $CO_2$) for 2 hours. Then, without removing the viral inoculum, the samples were transferred to 25 ml bottles and

4

supplied with 6 ml of RPMI +10% by volume FCS. Enzyme-linked immunosorbent assay (ELISA) for the HIV-1 p24 antigen was then effected of the cell culture supernatants using a NEN (Trade Mark) kit ( Du Pont).

## RESULTS

The results are shown below. The values in the second to sixth columns are amounts of p24, expressed as ng/ml.

| DAYS POST INFECTION | EXPERIMENT | | | | |
|---|---|---|---|---|---|
| | A | B | C | D | E |
| 7 | 0.24 | 0.06 | 0.13 | 0.02 | 0.19 |
| 13 | 0.016 | 0.02 | 0.07 | 0.10 | 0.07 |
| 20 | 1.70 | 0.05 | 0.03 | 0.08 | 0.07 |
| 33 | 0.08 | 0.12 | 0.02 | 1.45 | 0.95 |

A: HIV-1 infection control

B: Cells kept in medium and 2% DMSO after infection

C: Cells preincubated in 2% DMSO for 5 days before infection, then kept in medium and 2% DMSO after infection.

D: Cells preincubated in 2% DMSO for 5 days before infection, then kept in medium without DMSO after infection.

E: Samples from cell cultures, inected with HIV-1 preincubated for 24 hours in 2% DMSO. The cells were kept in medium without DMSO before and after infection.

## EXAMPLE 5: DMSO in vitro against HIV-1 CELLS AND VIRUS.

LDV/7, and EBV-nonproducer mixed T/B cell line (Viza et al, Biol Cell 1982 46 p1-10), MOLT-4 a human leukaemic T-cell line (Minowada et al, J.Nat. Cancer Inst. 1972 49 891-895) and H9, a T-cell line (Popovic et al Science 1984 224 497-500) were used as target cells. The cells were cultured in RPMI-1640 medium supplemented with 10% foetal calf serum without antibiotics.

The HIV strain used was HTLV-III -DA/1. The virus was grown either in H9 or LDV/7 cell cultures and concentrated cell-free virus was used for infecting the target cells.

## VIRUS INFECTION

Approximately $1 \times 10^5$ target cells were suspended in 1 ml of concentrated cell-free virus in RPMI medium without serum and incubated at $37°C$ for 60 minutes. The cell-virus suspension was subsequently diluted five times with RPMI medium and washed twice by centrifugation to remove the non-adsorbed virus. The cell pellet was resuspended in 6 ml of RPMI-1640 with 10% foetal calf serum and equally divided into three wells from a COSTAR cluster tissue culture tray that was kept at $37°C$ in 5% $CO_2$. The culture medium was changed every 4 days.

## DMSO

Dimethylsulfoxide research grade, anhydrous 99 + % obtained either from SERVA-Feinbiochemica, Heidelberg, FRG, or Aldrich-Chimie, France, was diluted in phosphate-buffered saline (PBS) and stored at 4°C until used, or was diluted directly into the tissue culture medium.

ASSAY OF HIV

The HIV-infected cells were monitored for the release of virus detected either as precipitable reverse transcriptase (RT) activity or HIV p24 core protein in cell culture supernatants assayed by a p24-specific ELISA system Dupont, NEK-041). Infected cells were also examined by indirect immunofluorescence analysis (IF) using MAbS to HTLV-III p24. The cell viability of infected and control cultures was periodically assessed microscopically by trypan blue exclusion.

ASSAYS FOR T-CELL SURFACE MARKERS

All cell lines used were tested for the presence of T-cell receptors by indirect immunofluorescence (IF) with MAbS OKT4, Leu/3A and OKT8 before and after infection with HIV, as well as before and after incubation in the presence of DMSO.

The results are given in Tables I-VII.

TABLE I

| p24 IN THE SUPERNATANTS OF HIV-INFECTED LDV/7 CELLS IN PRESENCE OF DMSO | | | | | | |
|---|---|---|---|---|---|---|
| POST-INFECTION DAY | 7 | 13 | 20 | 26 | 33 | 42 |
| INFECTION CONTROL | 0.045 | 0.08 | 1.07 | 1.36 | 0.23 | 0.68 |
| 2% DMSO FROM D.3 | - | - | - | 0.06 | - | - |
| 2% DMSO FROM D.-4 | - | - | - | - | 0.10 | 0.15 |
| 2% DMSO FROM D.-4 TO D.0 | - | - | 0.03 | N.D. | 1.15 | 0.78 |
| POST-INFECTION DAY | 7 | 15 | 23 | 31 | 38 | 52 |
| INFECTION CONTROL | 0.042 | 0.276 | 0.28 | 0.49 | 0.49 | 0.32 |
| 1% DMSO FROM D.-7 | 0.030 | 0.28 | 0.28 | 0.50 | 0.508 | 0.32 |
| N.D. indicates not done. - indicates that no p24 was detected. The Values of p24 are in ng/ml of TCM. | | | | | | |

TABLE II

| p24 IN THE SUPERNATANT OF HIV-INFECTED LDV/7 CELLS IN THE PRESENCE OF 2% DMSO FROM D.45 TO D.62 | | | | | | |
|---|---|---|---|---|---|---|
| POST-INFECTION DAY | 38 | 52 | 62 | 72 | 90 | 105 |
| INFECTION CONTROL | 0.49 | 0.32 | 0.52 | 0.85 | 0.47 | 0.50 |
| 2% DMSO FROM D.45 to D.62 | 0.49 | - | - | - | 0.46 | 0.51 |
| The values of p24 are in ng/ml of TCM. | | | | | | |

TABLE III

| EFFECT OF DMSO ON THE AMOUNT OF p24 IN SUPERNATANTS OF HIV-INFECTED MOLT-4 CELLS | | | | | | | |
|---|---|---|---|---|---|---|---|
| POST-INFECTION DAY | 5 | 19 | 27 | 39* | 46 | 53 | 59 |
| INFECTION CONTROL 2% DMSO | 0.48 0.34 | 0.51 0.024 | 0.50 - | 0.49 - | 0.50 - | 0.50 - | >0.50 - |

* DMSO was present in the culture medium from D.3 to D.39 post-infection. From D.39 onwards, DMSO was removed from the culture medium.
Cell viability ≥60% in all cultures.
The values of the p24 are expressed in ng/ml.

TABLE IV

| EFFECT OF DMSO ON RT ACTIVITY OF HIV-INFECTED LDV/7 CELLS | | | | | | | | | | |
|---|---|---|---|---|---|---|---|---|---|---|
| POST-INFECTION DAY | 4 | | 8 | | 11 | | 17 | | 30 | |
| | CPM | % C. | CPM | % C. | CPM | % C. | CPM | % C. | CPM | % C. |
| INFECTION CONTROL 2% DMSO | 1,145,674 899,525 | 100 78.5 | 558,347 129,857 | 100 32 | 245,832 78,644 | 100 32 | 355,615 62,562 | 100 18 | 171,827 8,328 | 100 4.8 |

%C. indicates percentage of RT activity of the control
Cell Viability ≥60%
DMSO was present in the tissue culture medium from Day O.

TABLE V

| EFFECT OF DMSO ON RT ACTIVITY OF HIV-INFECTED H9 CELLS | | | | | | | | | | |
|---|---|---|---|---|---|---|---|---|---|---|
| POST-INFECTION DAY | 4 | | 8 | | 11 | | 17 | | 30 | |
| | CPM | % C. | CPM | % C. | CPM | % C. | CPM | % C. | CPM | % C. |
| INFECTION CONTROL 2% DMSO | 3,403,260 2,039,377 | 100 60 | 675,621 195,711 | 100 29 | 629,999 131,419 | 100 20.8 | 187,907 53,828 | 100 28.6 | 70,646 6,082 | 100 8.6 |

Cell viability ≥60%
DMSO was present in the tissue culture medium from Day 0.

TABLE VI

| PERCENTAGE OF CD4[+] - LDV/7 CELLS AFTER 7 DAYS OF INCUBATION IN PRESENCE OF DMSO | |
|---|---|
| CONTROL | 9.1 |
| 2% DMSO | 8.7 |

In each case, the number of CD4[+] cells is within the expected % (11 ± 3%) characteristic of the LDV/7 cell line. 500 cells were counted in each case.

TABLE VII

| IMMUNOFLUORESENCE OF HIV-p24 IN LDV/7 CELLS TREATED WITH DMSO | | | | |
|---|---|---|---|---|
| POST-INFECTION DAY | 6 | 11 | 20 | 26 |
| INFECTION CONTROL 2% DMSO | 5.3 6.2 | 3.7 24.4 | 4.0 30.2 | 2.5 35 |

The values correspond to % of p24[+] cells as estimated by indirect immunofluorescence with BT3 Mab. 500 cells were counted in each case.

TABLE VIII

| IMMUNOFLUORESCENCE ON POST-INFECTION d.60 OF HIV-p24 IN MOLT-24 CELLS TREATED WITH DMSO | |
|---|---|
| INFECTION CONTROL | 4.5 |
| 2% DMSO | 13 |

The values correspond to % of p24[+] cells estimated on D.60 post-infection by indirect immunofluorescence with BT3 Mab. 500 cells were counted in each case.

Continuous incubation with 2% DMSO of LDV/7 HIV-infected cells inhibits the shedding of viral protein p24 into the tissue culture medium (TCM). This inhibition is quasi-total, and it is obtained equally whether DMSO is present from day 3 post-viral infection or from day 4 prior to infection. However, a four-day incubation preceding the HIV infection produces only a transient inhibitory effect. (Table I)

DMSO at 1% does not inhibit replication : the amounts of p24 in the supernatants are comparable to that of the control (Table 1). Incubation of HIV-infected LDV/7 cells with 2% of DMSO for 16 days produced a transient inhibition of p24 shedding into the TCM (Table II).

Inhibition of the RT activity in the supernatants of HIV-infected LDV/7 and H-9 cells is shown on Tables IV and V. DMSO produced almost total inhibition of the RT activity.

Immunofluorescence studies showed that incubation with DMSO does not prevent the expression of CD4 receptors on the surface of non-infected cells (Table VI) nor the expression of p24 antigen on the surface of HIV-infected cells (Tables VII and VIII).

DMSO inhibits viral replication, as is shown by the amounts of p24 protein or by the RT activity detected in TCM of HIV-infected cells grown in medium containing DMSO. This inhibition is dose-dependent : while 2% of DMSO is sufficient to produce inhibition, 1% has no effect.

Different cell lines used will withstand different concentrations of DMSO. Similarly, the amount necessary to inhibit viral replication varies from one cell line to the other. This suggests that the control mechanisms involved may be cellular rather than viral. By modifying the molecular environment of the virus, the virus in turn may modify its own regulatory mechanisms and slow or arrest the cycle of the viral replication. This could explain the fact that p24 is still present in the infected cell but viral production is arrested.

The fact that viral antigens are expressed on the cell surface may have implications for the potential clinical use of these compounds. If viral replication were controlled for a long period, this may allow the immune system to mount an effective immune response and eliminate the cells harboring the quiescent virus.

## Claims

1. The use of a compound of formula (I):

$$(R)_2 S(O)_n \qquad (I)$$

wherein R is methyl or ethyl and n is 1 or 2, in the preparation of a medicament for use in the treatment of an infection attributable to a retrovirus.

2. The use as claimed in claim 1 wherein the compound of formula (I) is dimethylsulphoxide.

3. The use as claimed in claim 1 or 2 wherein the medicament is for use in the treatment of an infection attributable to HTLV-1, HTLV-2 or a lentivirus.

4. The use as claimed in claim 3 wherein the medicament is for use in the treatment of an infection attributable to HIV-1, HIV-2, SIV or medi-visna.